# EUROPÄISCHE PATENTANMELDUNG

(11) **EP 2 612 622 A1**
(43) Veröffentlichungstag der Anmeldung: **10.07.2013**
(21) Anmeldenummer: 12192384.1
(22) Anmeldetag: 13.11.2012
(51) Int. Cl.: A61F 2/966

(54) **Medizinisches Implantat**

(30) Priorität: 04.01.2012 US 201261582835 P
(71) Anmelder: Biotronik AG, 8180 Bülach (CH)
(72) Erfinder: Fargahi, Amir, 8180 Bülach (CH)
(74) Vertreter: Lindner-Vogt, Karin L.

(57) **Zusammenfassung**

Die Erfindung geht aus von einer Vorrichtung (100) mit einem medizinischen Implantat (30), insbesondere einem Stent, zur Implantation in einen tierischen und/oder menschlichen Körper, wobei das medizinische Implantat (30) zur Positionierung an seinem Bestimmungsort an einem distalen Ende (16) eines Trägers (10) angeordnet und das medizinische Implantat (30) wenigstens zeitweise von einer Außenhülle (20) umgeben ist. Es wird vorgeschlagen, dass die Außenhülle (20) am distalen Ende (18) des Trägers (10) lösbar am Träger (10) festlegbar oder festgelegt ist.

## Beschreibung

Die Erfindung betrifft ein medizinisches Implantat, insbesondere einen Stent, zur Implantation in einen tierischen und/oder menschlichen Körper nach dem Oberbegriff des Patentanspruchs 1.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Es ist bekannt, dass Stents, die mit Kathetern verbunden sind, durch thermomechanische Belastung des Katheders in ihrer Transportverpackung verfrüht freigesetzt werden können. Um eine solche verfrühte Freisetzung zu verhindern, ist es bekannt, Katheter im proximalen Bereich, der dem Anwender zugewandt ist, zu arretieren. In diesem Fall wirkt die thermomechanische Belastung allerdings konzentriert auf den distalen Bereich, an dem der Stent angeordnet ist, so dass es trotzdem zu einer verfrühten Freisetzung kommen kann.

Alternativ wird auch eine Verlängerung des Abstands zwischen dem distalen Stent-Ende und dem Ende der Schutzhülle, welche den Stent bei Transport und Einführung an den Bestimmungsort schützt, vorgenommen. Die Größe des Abstands ist allerdings begrenzt. Je größer dieser Abstand ist, desto steifer und unflexibler wird der Katheter im distalen Bereich. Weiterhin erschwert dies die Positionierung des Stents im Einsatzgebiet.

Der Erfindung liegt die Aufgabe zugrunde, eine Vorrichtung mit einem medizinischen Implantat bereitzustellen, bei dem die Gefahr einer vorzeitigen Freisetzung des medizinischen Implantats verringert werden kann.

Die Aufgabe wird erfindungsgemäß durch die Merkmale des Patentanspruchs 1 gelöst. Günstige Ausgestaltungen und Vorteile der Erfindung ergeben sich aus den weiteren Ansprüchen, der Zeichnung und der Beschreibung.

Die Erfindung geht aus von einer Vorrichtung mit einem medizinischen Implantat, insbesondere einem Stent, zur Implantation im tierischen und/oder menschlichen Körper, wobei das medizinische Implantat zur Positionierung an seinem Bestimmungsort an einem distalen Ende eines Trägers angeordnet und das medizinische Implantat wenigstens zeitweise von einer Außenhülle umgeben ist,

Es wird vorgeschlagen, dass die Außenhülle am distalen Ende des Trägers lösbar am Träger festlegbar oder festgelegt ist.

Vorteilhaft wird erreicht, dass die Außenhülle sich nicht ungewollt vom Träger bzw. vom abgedeckten medizinischen Implantat lösen kann. Eine relative Bewegung der Außenhülle gegenüber dem Träger wird zuverlässig unterbunden. Vorteilhaft ist die Außenhülle so festgelegt oder ist so festlegbar, dass die so gebildete Verbindung am distalen Ende zwischen Außenhülle und Träger mindestens einer Zugbelastung standhält, die bei der Freisetzung des medizinischen Implantats an seinem Bestimmungsort ausgeübt wird. Eine typische Zugkraft hierzu liegt bei einigen Newton, z.B. 5 Newton. Ein ungewolltes Trennen von Außenhülle und Träger, beispielsweise in einer Transportverpackung, kann auf diese Weise vermieden werden.

Unter "festlegbar" soll insbesondere speziell ausgestattet, ausgelegt und/oder vorbereitet verstanden werden.

In diesem Zusammenhang soll unter einem "Implantat" insbesondere ein Körper verstanden werden, der zumindest eine Ersatzfunktion permanent oder für einen längeren Zeitraum bei Implantation in einen tierischen und/oder menschlichen Körper erfüllt. Denkbar wären hier sämtliche, dem Fachmann als zweckdienlich erscheinenden medizinischen Implantaten. Ein weiteres Beispiel ist ein Katheter mit selbstexpandierbarer Herzklappe.

Ferner soll in diesem Zusammenhang unter einem "Stent" insbesondere eine Struktur, wie beispielsweise ein Drahtgeflecht, verstanden werden, welches im Wesentlichen eine Gestalt und/oder eine Form des Stents und/oder den Stent selbst bildet und vorzugsweise aktiv expandierbar ist. Zudem ist der Stent vorzugsweise aus einem elastischen oder superelastischen Material, etwa einem metallischen Material und/oder aus einer Kombination von mehreren metallischen Materialien gefertigt, wie beispielsweise Eisen, Magnesium, Nickel, Wolfram, Titan, Zirkonium, Niob, Tantal, Zink, Silizium, Lithium, Natrium, Kalium, Kalzium, Mangan und/oder jedem anderen, dem Fachmann als sinnvoll erscheinenden Material.

Unter der Wendung "aktiv expandierbar" soll insbesondere verstanden werden, dass der Bereich selbständig bzw. selbsttätig, also ohne fremde Hilfe expandiert bzw. expandierbar ist. Vorteilhafterweise ist der aktiv expandierbare Bereich aus einem Formgedächtnis-Material, wie beispielsweise eine Kupfer-Zink-Aluminium-Legierung und/oder Nickel-Titan-Legierung, vorzugsweise Nitinol, gefertigt. Zudem wäre es auch denkbar, dass zwei aktiv expandierbare Bereiche vorgesehen sind, die gleiche oder unterschiedliche Expansionscharakteristika aufweisen. Unter "passiv expandierbar" soll insbesondere verstanden werden, dass der Bereich unselbständig und/oder mittels einer von außen zugeführten Kraft expandierbar bzw. plastisch verformbar ist. Eine Expansion mit Hilfe eines selbstexpandierbaren Materials und/oder durch den ersten Bereich soll hier insbesondere nicht verstanden werden. Konstruktiv einfach kann die passive Expansion mittels eines Ballonkatheters erfolgen. Ferner kann es vorteilhaft sein, wenn der passiv expandierbare Bereich bzw. der Stent zumindest Kobalt und/oder Chrom aufweist, vorzugsweise in der Form von Edelstahl bzw. medizinischem Edelstahl und/oder eines Cr-Ni-Fe-Stahl - hier bevorzugt die Legierung 316L - oder einem Co-Cr-Stahl.

Unter "distalem Ende des Trägers" soll insbesondere dasjenige Ende des Trägers verstanden werden, welches in den menschlichen oder tierischen Körper eingeführt werden soll, um das medizinische Implantat an seinen Bestimmungsort zu bringen. Das entgegengesetzte, proximale Ende des Trägers ist dem Anwender zugewandt und bleibt außerhalb des menschlichen oder tierischen Körpers.

Der Träger kann ein flexibler Schaft oder Schlauch sein, der in seinem Inneren beispielsweise einen Führungsdraht sowie ein oder mehrere Lumen für Gas oder Flüssigkeit aufweisen kann. Insbesondere kann der Träger als Katheter oder als Innenschaft eines Katheters ausgebildet sein.

Unter "lösbar am Träger festlegbar oder festgelegt" ist insbesondere zu verstehen, dass ein Trennen von Außenhülle und Träger vorzugsweise werkzeuglos erfolgen kann und insbesondere eine Beschädigung von Außenhülle und/oder Träger beim Lösen der Außenhülle von dem Träger unter normalen Betriebsbedingungen ausgeschlossen ist.

Vorteilhaft kann die Außenhülle mit einem distalen Bereich des Trägers kraftschlüssig oder formschlüssig verbunden sein. Dies ermöglicht eine axiale Sicherung der Außenhülle auf dem Träger.

Gemäß einer günstigen Ausgestaltung kann die Außenhülle im distalen Bereich wenigstens eine Bohrung quer zu einer Längsachse des Trägers aufweisen, die mit einer Bohrung des Trägers fluchtet. Vorteilhaft kann ein Verbindungselement durch die wenigstens eine Bohrung der Außenhülle und die wenigstens eine Bohrung des Trägers geführt oder führbar sein. Vorzugsweise kann das Verbindungselement ein Drahtstück sein.

Günstigerweise kann das Drahtstück einen Durchmesser aufweisen, der kleiner ist als der Durchmesser eines Führungsdrahts ist, der im Träger zur Einführung des medizinischen Implantats an seinem Bestimmungsort vorgesehen sein kann. Unter "vorgesehen" soll insbesondere speziell ausgestattet, ausgelegt und/oder vorbereitet verstanden werden. Beispielsweise kann der Durchmesser des Drahtstücks nur etwas halb so dick sein wie der Führungsdraht. Das Material des Drahtstücks kann metallisch oder nichtmetallisch sein. Beispielsweise kann das Drahtstück aus Edelstahl oder Nitinol gebildet sein, oder aus einem Kunststoff wie z.B. Polyamid (PA), Polyaramid (z.B. Kevlar von Firma DuPont), Polyethylen (PE), Polyethylenterephthalat (PET) und dergleichen sein.

Alternativ oder zusätzlich kann die Außenhülle im distalen Bereich am Träger festgeklemmt oder festklemmbar sein.

Unter "festgeklemmt" oder "festklemmbar" soll verstanden werden, dass die Außenhülle in radialer Richtung auf den Träger gepresst wird oder pressbar ist. Dies kann umlaufend am Umfang der Außenhülle erfolgen oder beispielsweise an zwei oder mehr sich am Träger gegenüberliegenden Bereichen der Außenhülle. Insbesondere soll unter "festlegbar" speziell ausgestattet, ausgelegt und/oder vorbereitet verstanden werden.

Die Außenhülle kann im distalen Bereich mit einer Klemmvorrichtung am Träger festgeklemmt oder festklemmbar sein. Die Klemmvorrichtung kann beispielsweise als elastisches Band ausgebildet sein, oder in Form einer Schlauchschelle ausgebildet sein, oder in Form einer Schnürung ausgebildet sein. Die Art der Klemmvorrichtung kann auf den speziellen Einsatzfall bzw. auf das jeweilige medizinische Implantat abgestimmt werden.

Vorteilhaft kann der Träger eine Einschnürung aufweisen, in deren Bereich die Außenhülle festgeklemmt oder festklemmbar ist. Damit kann der geklemmte Bereich der Außenhülle axial am Träger gesichert werden. Dabei können auch vorhandene Einschnürungen am Träger verwendet werden, so dass der Träger praktisch nicht verändert werden muss.

Die Erfindung ist nachfolgend beispielhaft, anhand von in Zeichnungen dargestellten Ausführungsbeispielen, näher erläutert. Es zeigen in schematischer Darstellung:
- Fig. 1: einen Ansicht eines selbstexpandierenden medizinischen Implantats auf einem Träger, welches aus seiner Außenhülle freigesetzt wurde;
- Fig. 2: ein erstes Ausführungsbeispiel einer Vorrichtung gemäß der Erfindung mit einem Sicherungsdraht;
- Fig. 3: ein weiteres Ausführungsbeispiel einer Vorrichtung gemäß der Erfindung mit einer Klemmvorrichtung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

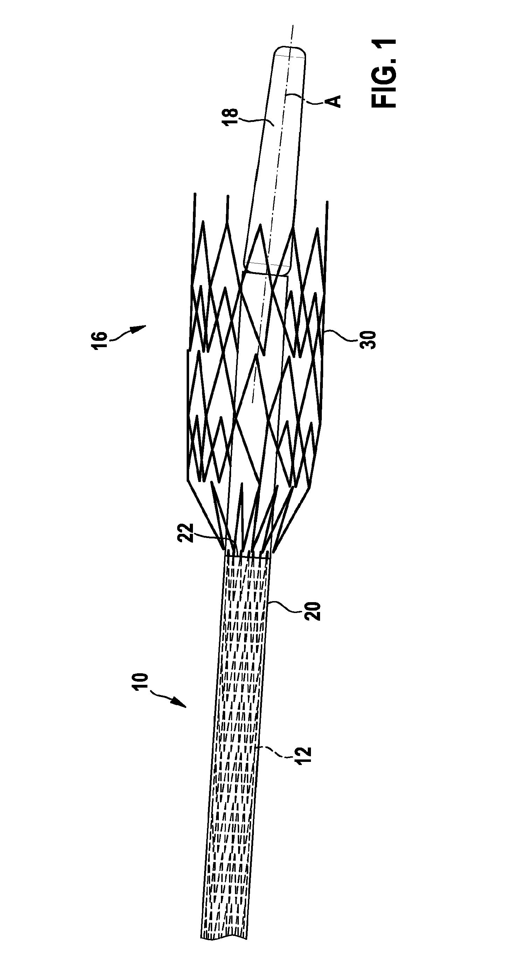

Fig. 1 zeigt ein Beispiel eines selbstexpandierenden medizinischen Implantats 10 in Form eines selbstexpandierenden Stents auf einem Träger 12 in Form eines Innenschafts eines Katheters mit einer Katheterspitze 18, wobei das medizinische Implantat 10 durch thermomechanische Belastung ungewollt aus seiner Außenhülle 20 freigesetzt wurde, da die Außenhülle 20 mit ihrer Abschlusskante 22 axial unter die Position des medizinischen Implantats 10 gerutscht ist. Das medizinische Implantat 30 hat sich aus seiner gekrimpten Position auf dem Träger 12, in der das medizinische Implantat 30 an seinen Bestimmungsort gebracht werden sollte, gelöst und hat sich aufgeweitet. Das medizinische Implantat 30 muss daher verworfen werden.

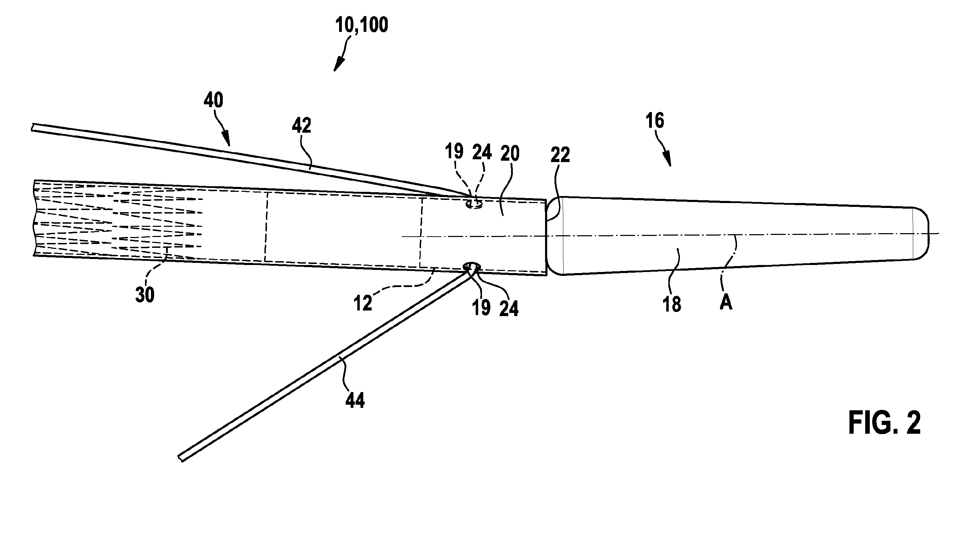

Die Fig. 2 zeigt eine Ausgestaltung einer Vorrichtung 100 nach der Erfindung mit einem medizinischen Implantat 30 in Form eines Stents, zur Implantation in einen tierischen und/oder menschlichen Körper. Das medizinische Implantat 30 ist zur Positionierung an seinem Bestimmungsort an einem distalen Ende 16 eines Trägers 10 in Form eines Innenschafts 12 eines Katheters angeordnet. Der Katheter weist an seiner starren Katheterspitze 18 eine Längsachse A auf, während der Innenschaft 12 weitgehend flexibel ausgebildet ist.

Das medizinische Implantat 30 ist zum Transport und zum Einführen an seinen Bestimmungsort einer Außenhülle 20 umgeben. Die Außenhülle 20 ist am distalen Ende 16 des Trägers 10 lösbar am Träger 10 festgelegt.

Im Ausführungsbeispiel der Fig. 2 ist die Außenhülle 20 im distalen Bereich 16 mit dem Innenschaft 12 durch ein Verbindungsmittel 40 verbunden, das durch korrespondierende Bohrungen 24 der Außenhülle 20 unterhalb deren Abschlusskante 22 und Bohrungen 19 im Innenschaft 12 quer zur Längsachse A des Trägers 10 geführt ist. Das Verbindungsmittel 40 ist hier als Drahtstück ausgeführt, das zu beiden Seiten mit je einem Ende 42, 44 übersteht. Die Außenhülle 20 ist jetzt gegen eine axiale Relativbewegung zum Innenschaft 12 gesichert.

Das Drahtstück (Verbindungsmittel 40) kann einen sehr geringen Durchmesser aufweisen, der im Bereich zwischen 100 µm und 200 µm liegen kann. Insbesondere ist der Durchmesser geringer als der eines üblichen Führungsdrahts, wie er in Kathetern vorgesehen ist, dessen Durchmesser typischerweise mehr als 400 µm beträgt. Die Bohrungen 24, 19 sind zweckmäßigerweise etwas größer als der Durchmesser des Drahtstücks (Verbindungsmittel 40), um ein einfaches Einfädeln des Drahtstücks 40 zu ermöglichen.

Die Enden 42, 44 des Drahtstücks (Verbindungsmittel 40) können mit einem leicht lösbaren Knoten oder durch Verdrillen der Enden 42, 44 miteinander verknüpft werden, um die Verbindung zu sichern. Das Drahtstück kann vom Anwender leicht entfernt werden.

Dadurch, dass das Verbindungsmittel 40 durch die Außenhülle 20 und den Innenschaft 12 geführt ist, muss dieses zwangsläufig erst entfernt werden, bevor der Führungsdraht in den Katheter eingesetzt werden kann.

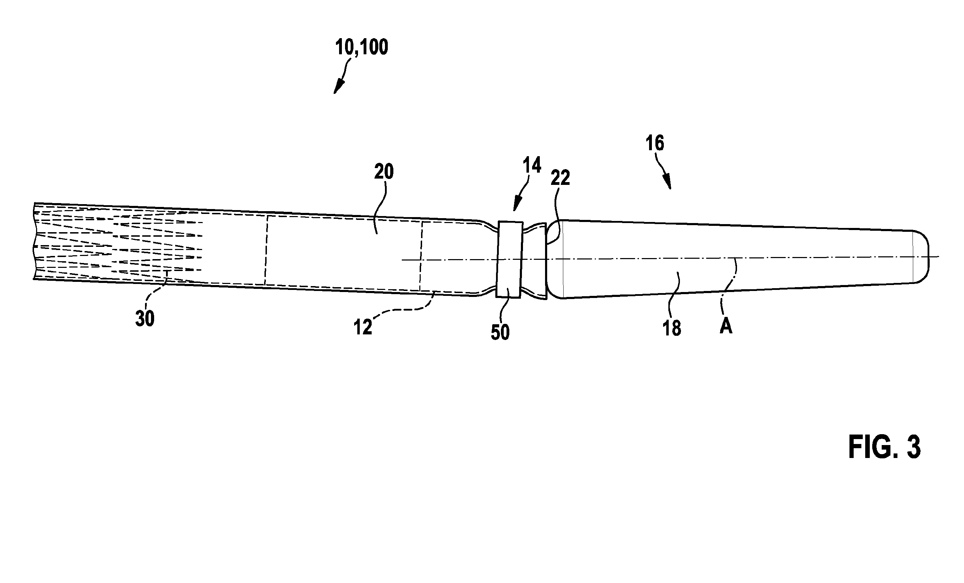

In einer alternative Ausgestaltung gemäß Fig. 3 ist die Außenhülle 20 im distalen Bereich 12 am Träger 10 festgeklemmt. Der grundlegende Aufbau der Vorrichtung 100 entspricht weitestgehend dem der Vorrichtung in Fig. 2, so dass zur Vermeidung unnötiger Wiederholungen auf die Figurenbeschreibung der Fig. 2 verwiesen wird.

Das Verbindungsmittel 40 der Fig. 2 ist hier durch eine Klemmvorrichtung 50 ersetzt, mit der die Außenhülle 20 im distalen Bereich 16 am Träger 10 festgeklemmt ist. Der Träger 10 bzw. der Innenschaft 12 weist eine Einschnürung 14 auf, in deren Bereich die Klemmvorrichtung 50 angeordnet sein kann.

Die Klemmvorrichtung 50 kann eine Drahtschlaufe sein oder eine Schlauchschelle oder eine Bandage.

Denkbar ist auch eine Klemmvorrichtung in der Art einer Klammer, welche die Katheterspitze 18 übergreift und die Außenhülle 20 auf dem Innenschaft 12 festklemmt.

Einige Beispiele für Materialien, welche für die Außenhülle 20 und den Innenschaft (Träger 10) mit so genanntem Monolayer-Design eingesetzt werden können, sind:
- für die Außenhülle 20 wenigstens eines der Materialien wie z.B.
   ABS (Acrylonitril-Butadiene-Styrol)
   ANS (Acrylonitril-Styrol)
   Delrin Acetal (ein ungefülltes Homopolymer, z.B. von der Firma DuPont)
   PVC (Polyvinylchlorid)
   PEN (Polyethylennaphtalat)
   PBT (Polybutylenterephtalat)
   PC (Polykarbonat)
   PEEK (Polyetheretherketon)
   PEI (Polyetherimid)
   PES (Polyethersulfon)
   PET (Polyethylenterephthalat)
   PETG (Polyethylenterephthalatglykol)
   PA (Polyamid)
   Polyether
   Polyester
   Kynar ADX (ein thermoplastischer fluorinierter funktionalisierter Polymer der Firma Arkema)
   PEBAXs (alle Härtegrade) (ein Polyether-Block-Amid der Firma Arkema) PTFE (Polytetrafluorethylen)
- für den Träger 10 wenigstens eines der Materialien wie z.B.
   PI (Polyimid)
   Hytrell (ein Polyesterelastomer-Blockcopolymer der Firma DuPont) PMMA (Polymethylmethacrylat)
   PUR (Polyurethan)
   EVA (Ethylenvinylacetat)
   Ethylenvinylalkohol eingebettet in PE (low, linear low, medium, high density Polyethylen)
   Latexgummi
   FEP (Perfluor (Ethylen-Propylen-) Kunststoff)
   TFE (Tetrafluorethylen)
   PFA (Perfluoralkoxylalkan)
   Polypropylen
   Polysiloxane
   Flüssigkristallpolymere
   Silikongummi
   SAN (Styrol-Akrylonitril)
   PA (Polyamid), insbesondere PA-6, -6.6, -6.10, -6.12, -11, -12)

Einige Beispiele für Materialien, welche für die Außenhülle 20 und den Innenschaft (Träger 10) mit so einem genannten Multilayer-Coextrudiertem Design eingesetzt werden können, sind:
- für die Außenschicht wenigstens eines der Materialien wie z.B.
   PI (Polyimid)
   PEBAXs (alle Härtegrade) (ein Polyether-Block-Amid der Firma Arkema)
   PA (Polyamid), insbesondere PA-6, -6.6, -6.10, -6.12, -11, -12)
- für die mittlere Schicht Materialien, die als Haftvermittler geeignet sind, d.h. eine Haftung zwischen Außen- und Innenschicht erzeugen, wie z.B.
   linear low density Polyethylen
- für die Innenschicht wenigstens eines der Materialien wie z.B.
   FEP (Perfluor-(Ethylen-Propylen-) Kunststoff)
   HDPE (high density Polyethylen)
   PTFE (Polytetrafluorethylen) Teflon

Ein solcher Katheter mit so genanntem Multilayer-coextrudiertem Design kann günstigerweise so aufgebaut sein, dass die Außenhülle und der Innenschaft (Träger 10) jeweils aus Außenschicht, mittlerer Schicht und Innenschicht bestehen.

Einige Beispiele für Materialien, welche für die Außenhülle 20 und den Innenschaft (Träger 10) mit einem so genannten Multilayer-coiled/braided-verstärktem Design eingesetzt werden können, sind:
- für die Außenschicht wenigstens eines der Materialien wie z.B.
   PI (Polyimid)
   PEBAXs (alle Härtegrade)
   PA (Polyamid), insbesondere PA-6, -6.6, -6.10, -6.12, -11, -12)
- für die braid/coil Schicht wenigstens eines der Materialien wie z.B.
   Nitinol-Draht (z.B. Runddraht, Flachdraht)
   Edelstahl-Draht (z.B. Runddraht, Flachdraht)
   Polyaramidfaser, z.B. Kevlar von Firma DuPont
- für die Innenschicht wenigstens eines der Materialien wie z.B.
   FEP (Perfluor-(Ethylen-Propylen-) Kunststoff)
   HDPE (high density PE)
   PTFE (Polytetrafluorethylen) Teflon

Ein solcher Katheter mit Multilayer-coiled/braided verstärktem Design kann beispielsweise so aufgebaut sein, dass die Außenhülle und der Innenschaft (Träger 10) jeweils aus Außenschicht, mittlerer Schicht und Innenschicht bestehen. Bei einem braid-verstärkten Design ist die mittleren Schicht mit einem in die mittlere Schicht integrierten Geflecht (braid) aus einer metallischen Faser oder Kunststoff-Faser oder Kohlefaser verstärkt oder die mittlere Schicht besteht aus einem solchen Geflecht. Bei einem coil-verstärkten Design ist die mittleren Schicht mit einer in die mittlere Schicht integrierten Spule (coil) aus einem metallischen Draht, der einen beliebigen Querschnitt aufweisen kann (z.B. Runddraht, Flachdraht etc. oder eine beliebige Kombination davon) oder Kunststoff-Faser oder Kohlefaser verstärkt, die mit einer oder mehreren Windungen zu einer Spule gewickelt sind, oder die mittlere Schicht besteht aus einer solchen Spule. Dabei kann die mittlere Schicht auch sowohl mit Geflecht als auch mit einer Spule verstärkt sein.

Einige Beispiele für Materialien, welche für die Außenhülle 20 und den Innenschaft (Träger 10) mit so genanntem Multilayer-braided-verstärktem Design eingesetzt werden können, sind:
- für die Außenschicht wenigstens eines der Materialien wie z.B.
   PI (Polyimid)
   PEBAXs (alle Härtegrade)
   PA (Polyamid), insbesondere PA-6, -6.6, -6.10, -6.12, -11, -12)
- für die braid-Schicht wenigstens eines der Materialien wie z.B.
   Nitinol-Draht (mit beliebigem Querschnitt, z.B. Runddraht, Flachdraht etc., oder eine beliebige Kombination davon)
   Edelstahl-Draht (mit beliebigem Querschnitt, z.B. Runddraht, Flachdraht etc., oder eine beliebige Kombination davon)
   Kobalt-Chrom-Legierungs-Draht (mit beliebigem Querschnitt, z.B. Runddraht, Flachdraht etc., oder eine beliebige Kombination davon)
   Polyaramidfaser (z.B. Kevlar von Firma DuPont)
- für die Innenschicht wenigstens eines der Materialien wie z.B.
   FEP (Perfluor-(Ethylen-Propylen-) Kunststoff)
   HDPE (high density PE)
   PTFE (Polytetrafluorethylen) Teflon

## Patentansprüche

1. Vorrichtung (100) mit einem medizinischen Implantat (30), insbesondere einem Stent, zur Implantation in einen tierischen und/oder menschlichen Körper, wobei das medizinische Implantat (30) zur Positionierung an seinem Bestimmungsort an einem distalen Bereich (16) eines Trägers (10) angeordnet und das medizinische Implantat (30) wenigstens zeitweise von einer Außenhülle (20) umgeben ist, **dadurch gekennzeichnet, dass** die Außenhülle (20) am distalen Bereich (16) des Trägers (10) lösbar am Träger (10) festlegbar oder festgelegt ist.

2. Vorrichtung nach Anspruch 1, **dadurch gekennzeichnet, dass** die Außenhülle (20) mit dem distalen Bereich (16) des Trägers (10) kraftschlüssig verbindbar oder verbunden ist.

3. Vorrichtung nach Anspruch 1, **dadurch gekennzeichnet, dass** die Außenhülle (20) mit dem distalen Bereich (16) des Trägers (10) formschlüssig verbindbar oder verbunden ist.

4. Vorrichtung nach einem der vorhergehenden Ansprüche, **dadurch gekennzeichnet, dass** die Außenhülle (20) im distalen Bereich (16) wenigstens eine Bohrung (24) quer zu einer Längsachse (A) des Trägers (10) aufweist, die mit einer Bohrung (19) des Trägers (10) fluchtet.

5. Vorrichtung nach Anspruch 4, **dadurch gekennzeichnet, dass** ein Verbindungselement (40) durch die wenigstens eine Bohrung (24) der Außenhülle (20) und die wenigstens eine Bohrung (19) des Trägers (10) führbar oder geführt ist.

6. Vorrichtung nach Anspruch 5, **dadurch gekennzeichnet, dass** das Verbindungselement (40) ein Draht ist.

7. Vorrichtung nach Anspruch 6, **dadurch gekennzeichnet, dass** der Draht einen Durchmesser aufweist, der kleiner ist als der Durchmesser eines Führungsdrahts ist, der im Träger (10) zur Einführung des medizinischen Implantats (30) an seinem Bestimmungsort vorgesehen ist.

8. Vorrichtung nach einem der vorhergehenden Ansprüche, **dadurch gekennzeichnet, dass** die Außenhülle (20) im distalen Bereich (16) am Träger (10) festgeklemmt oder festklemmbar ist.

9. Vorrichtung nach Anspruch 8, **dadurch gekennzeichnet, dass** die Außenhülle (20) im distalen Bereich (16) mit einer Klemmvorrichtung (50) am Träger (10) festgeklemmt oder festklemmbar ist.

10. Vorrichtung nach einem Ansprüche 8 oder 9, **dadurch gekennzeichnet, dass** der Träger (10) eine Einschnürung (14) aufweist, in deren Bereich die Außenhülle (20) am Träger (10) festgeklemmt oder festklemmbar ist.
